(19) Europäisches Patentamt
European Patent Office
Office européen des brevets

(11) Publication number: 0 177 327 B1

# (12) EUROPEAN PATENT SPECIFICATION

(45) Date of publication of patent specification: 24.01.90

(51) Int. Cl.$^5$: C 07 C 2/84, B 01 J 23/00

(21) Application number: 85306994.6

(22) Date of filing: 01.10.85

## (54) Upgrading low molecular weight alkanes.

(30) Priority: 02.10.84 US 657095

(43) Date of publication of application:
09.04.86 Bulletin 86/15

(45) Publication of the grant of the patent:
24.01.90 Bulletin 90/04

(84) Designated Contracting States:
DE FR GB IT NL

(56) References cited:
DE-A-3 237 079
US-A-4 443 645
US-A-4 443 649
US-A-4 495 374
US-A-4 499 322
US-A-4 499 324

J.C. BAILER et al:"Comprehensive Inorganic Chemistry" (1973) p. 67,124

(73) Proprietor: THE STANDARD OIL COMPANY
Midland Building
Cleveland, Ohio 44115 (US)

(72) Inventor: Brazdil, James F.
916 Aintree Park Dr.
Mayfield Village Ohio 44143 (US)
Inventor: Grasselli, Robert K.
462-2 Overlook Drive
Aurora Ohio 44202 (US)
Inventor: Bartek, Joseph P.
945 Eastlawn Dr.
Highland Heights Ohio 44143 (US)

(74) Representative: Smith, Sydney et al
Elkington and Fife Beacon House 113 Kingsway
London WC2B 6PP (GB)

EP 0 177 327 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the invention

This invention relates to upgrading low molecular weight alkanes to form higher order hydrocarbons. More specifically, the present invention is directed to a vapor phase reaction of low molecular weight alkanes in the presence of novel catalyst formulations to synthesize higher molecular weight hydrocarbons, especially the reaction of methane to synthesize ethane and ethylene. The higher order hydrocarbons may then be subsequently reacted to form predominantly liquid hydrocarbon products.

Background of the invention

The predominant source of methane, an abundant low molecular weight alkane, is natural gas, which is found in porous reservoirs generally associated with crude oil reserves. From this source comes most of the natural gas used for heating purposes. Quantities of natural gas are also known to be present in coal deposits and are by-products of crude oil refinery processes and bacterial decomposition of organic matter. Natural gas obtained from these sources is generally utilized as a fuel at the site.

Prior to commercial use, natural gas must be processed to remove water vapor, condensible hydrocarbons and inert or poisonous constituents. Condensible hydrocarbons are generally removed by cooling natural gas to a low temperature and then washing the natural gas with a cold hydrocarbon liquid to absorb the condensible hydrocarbons, which comprise ethane and heavier hydrocarbons. Such treating of natural gas can occur at the wellhead or at a central processing station. The processed natural gas comprises predominantly methane, and minor quantities of ethane, propane, butane, pentane, carbon dioxide and nitrogen. Generally, processed natural gas comprises from about 50 volume percent to more than 95 volume percent methane.

Natural gas is used principally as a source of heat in residential, commercial and industrial service. Methane also has commercial uses in the chemical processing industry. The largest use for methane, other than as a primary fuel, is in the production of ammonia and methanol. Ammonia is a basic ingredient of fertilizers and is also a common feedstock in the production of petrochemicals such as acrylonitrile and nylon-6. Methanol is a precursor material for products such as formaldehyde, acetic acid and polyesters. Methane has also been used as a feedstock for the production of acetylene by electric-arc or partial-oxidation processes. Another commercial use for methane is in the production of halogenated products such as methyl chloride, methylene chloride, chloroform and carbon tetrachloride. Methane also reacts with ammonia to produce hydrogen cyanide.

Most processed natural gas is distributed primarily through an extensive pipeline distribution network. As gas reserves in close proximity to gas usage decrease, new sources that are secured require much additional transportation. Such distant sources may not be amenable to transport by pipeline, such as sources that are located in areas requiring economically unfeasible pipeline networks or in areas requiring transport across large bodies of water. This concern has been addressed in several ways. One such solution has been to build a production facility at the site of the natural gas deposit to manufacture one specific product. This approach is limited as the natural gas can be used only for one product, pre-empting other feasible uses for methane. Another approach has been to liquify methane and transport the liquid methane in specially designed tanker ships. Natural gas can be reduced to 1/600th of the volume occupied in the gaseous state by such cryogenic processing, and with proper procedures, safely stored or transported. The processes for liquifying natural gas to a temperature of about −162°C, transporting, and revaporizing are complex and energy intensive.

Still another approach has been the conversion of methane to higher order hydrocarbon products that can be easily handled and transported, preferably hydrocarbon products that exist in a liquid state. The conversion of methane to higher order hydrocarbons, especially ethane and ethylene, would retain the material's versatility for use as precursor materials in chemical processing. Known dehydrogenation and polymerization processes are available for further conversion of ethane and ethylene to liquid hydrocarbons, such as the process taught by Chu in United States Patent 4,120,910 and Chen et al in United States patent 4,100,218, both disclosures being incorporated herein by reference. In these ways, easily transportable commodities may be derived directly from natural gas at the wellhead. The drawback to implementing such a process, however, has been in obtaining a sufficient conversion rate of methane to higher order hydrocarbons.

The conversion of methane to higher molecular weight hydrocarbons at high temperatures, in excess of 1,000°C is well-known. This process, however, is very energy intensive, and has not been developed to the point where high yields are obtained even with the use of catalysts. Known catalysts, such as chlorine, are also typified as extremely corrosive at operating conditions.

The catalytic oxidative coupling of methane at atmospheric pressure and temperatures of from about 500° to 1,000°C have been investigated by several researchers. G. E. Keller and M. M. Bhasin reported the synthesis of ethylene via oxidative coupling of methane over a wide variety of metal oxides supported on an alpha alumina structure, *Journal of Catalysis* 73, 9—19 (1982). This article discloses the use of single component oxide catalysts that exhibited methane conversion to higher order hydrocarbons at rates no greater than 4 percent. The process by which Keller and Bhasin oxidized methane was cyclic, varying the feed composition between methane, nitrogen and air (oxygen) to obtain higher selectivities.

West German Patent DE 3237079 to Baerns and Hinsen report also the use of single supported component oxide catalysts. The process taught by Baerns and Hinsen utilizes low oxygen partial pressure to give a high selectivity for the formation of ethane and ethylene. The conversion of methane to such desired products however remains low, on the order of from about four to about seven percent conversion.

United States Patents 4,443,644—4,443,649 inclusive to Jones et al. teach a method for synthesizing hydrocarbons from methane which comprises contacting methane with a single component metal oxide at a temperature between about 500° and 1,000°C. The process taught by Jones, et al. in these disclosures produces high selectivity to higher order hydrocarbons but at very low conversion rates, on the order of less than 4 percent overall conversion to higher order hydrocarbons. In addition to synthesizing hydrocarbons, Jones et al. also produce a reduced metal oxide. This reaction promoter must be frequently regenerated by contact with oxygen. Jones et al. teach that the preferred process entails physically separate zones for a methane contacting step and for an oxygen contacting step, with the reaction promoter recirculating between the two zones.

What is lacking in the area of natural gas conversion to higher order hydrocarbon products is a direct process for such conversion having a high selectivity for the formation of hydrocarbon products and also at commercially feasible methane conversion rates.

It is therefore one object of the present invention to provide a process to upgrade low molecular weight alkanes to produce higher order hydrocarbons with high selectivity and conversion rates.

It is a further object of the present invention to provide a process to upgrade methane to produce predominantly ethane and ethylene.

It is still another object of the present invention to provide novel catalyst compositions useful in the upgrading of low molecular weight alkanes to produce higher order hydrocarbon products.

Yet another object of this invention is to provide a process to upgrade low molecular weight alkanes to predominantly liquid hydrocarbon products.

Additional objects of the invention will become apparent to those skilled in the art in the following description of the invention and in the appended claims.

Summary of the invention

In general, the invention relates to a process for the conversion of low molecular weight alkanes to higher order hydrocarbons comprising contacting the low molecular weight alkane at a reaction temperature of from 500°C to 1000°C with a catalyst of the formula

$$A_a B_b C_c O_x$$

wherein
A is Pb, Bi, Sn, Sb, Tl, In, Mn, Cd, Ge or mixtures thereof;
B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and
x is the number of oxygens needed to fulfill the valence requirements of the other elements.

The present invention also includes novel catalysts for the conversion of low molecular weight alkanes to higher order hydrocarbons of the formula

$$A_a B_b C_c O_x$$

wherein
A is Pb, Bi, Sn, Sb, Tl, In, Mn, Cd, Ge or mixtures thereof;
B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and
xi is the number of oxygens needed to fulfill the valence requirements of the other elements; which catalysts has been heat treated at a temperature of between 500°C and 1200°C.

The invention also pertains to a process for upgrading low molecular weight alkanes to substantially liquid hydrocarbon products, which process comprises

a) contacting low molecular weight alkanes at a reaction temperature of from 500°C to 1000°C in the presence of a catalyst of the formula

$$A_a B_b C_c O_x$$

wherein
A is Pb, Bi, Sn, Sb, Tl, In, Mn, Cd, Ge or mixtures thereof;

B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and
x is the number of oxygens needed to fulfill the valence requirements of the other elements so as to synthesize a higher order hydrocarbon product effluent; and

b) dehydrogenating said higher order hydrocarbon product effluent, and/or contacting said effluent with a catalyst so as to yield substantially liquid hydrocarbon products.

The invention especially pertains to upgrading methane to predominantly ethane and ethylene and to their subsequent conversion to liquid hydrocarbon products.

Detailed description of the invention

As used herein, the phrase "low molecular weight alkanes" refers to alkanes of the formula $C_nH_{2n+2}$ wherein n ranges from 1 to 5. Thus, lower molecular weight alkanes include methane, ethane, propane, butane and pentane, each a major or minor constituent found in natural gas. The phrase "higher order hydrocarbons" refers to the product of a reaction wherein an alkane is reacted to yield a hydrocarbon having at least one carbon atom more than that found in the reactant alkane. The phrase "substantially liquid hydrocarbon products", as used herein, refers to hydrocarbons that exist primarily in the liquid state at about 25°C and about one atmosphere.

In the process of the present invention a low molecular weight alkane is contacted in the presence of an oxidative catalyst in the vapor phase to form higher order hydrocarbons, and subsequently may be further processed if necessary to yield substantially liquid hydrocarbon products. The oxidative catalyst comprises a novel multicomponent formulation.

Advantages over previously disclosed processes for the conversion of low molecular weight alkanes include higher selectivities to higher molecular weight hydrocarbons, higher conversion rates, longer catalyst life and avoidance of corrosive and/or expensive gas phase promoters. Additionally, the process of the present invention is well-suited to a continuous process and also useful in the cyclic requirements of several known processes.

One low molecular weight alkane of special interest is methane. Methane, in the form of natural gas, may also have associated with it minor amounts of other hydrocarbons such as ethane, propane, butane, and pentane, water, carbon dioxide; nitrogen; carbon monoxide and inert gases. The presence of such gas phase additives does not affect the efficiency of the present process. Indeed, the process disclosed herein is effective for upgrading not only methane, but also the other low molecular weight alkanes found in natural gas. Although reference herein below may be directed to the upgrading of methane, it is understood that the same remarks are applicable for other low molecular weight alkanes.

The process of the present invention is an oxidative reaction. Gaseous oxygen may be provided as substantially pure oxygen or diluted with nitrogen, carbon dioxide, or other inert gases, or may be provided in air. Alternatively, the reaction may occur in the substantial absence of gaseous oxygen, oxygen for the reaction then being derived almost entirely from the oxygen in the multicomponent catalyst employed in the reaction.

A reaction feed stream comprises from about 15 volume percent to about 100 volume percent methane and from zero volume percent to 50 volume percent oxygen. A diluent gas may also be present in the reaction feed stream.

The process of the present invention is carried out by contacting the gaseous reactants, predominantly methane and oxygen, with one of the novel catalysts described below in a suitable fluid bed reactor, fixed bed reactor or any other suitable reactor configuration such as a moving bed reactor, swing reactor system or membrane reactor. The reaction can be conducted continuously or in a batch-type mode. The reaction temperature should be maintained in the range of from 500°C to 1,000°C, and preferably between 600°C and 800°C.

The contact time of the reactants with the catalyst is from 0.05 seconds to 20 seconds, and preferably from 0.1 seconds to 2 seconds.

The novel catalysts provided by the present invention are believed to be multicomponent oxide complexes and comprise compositions described by the empirical formula

$$A_aB_bC_cO_x$$

wherein
A is Pb, Bi, Sn, Sb, Tl, In, Mn, Cd, Ge or mixtures thereof;
B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and

x is the number of oxygens needed to fulfill the valence requirements of the other elements.

A is preferably Pb, Bi, Sn, Sb, Mn or mixtures thereof, and most preferably is Pb. B is preferably Mg, Cu, Cr, Ca, Zn, La or mixtures thereof. Most preferably, B is Mg, Ca, Zn or mixtures thereof. Preferably the value of a is from 0.1 to 10, the value of b is from 0.1 to 10, and the value of C is from 0 to 10.

The catalyst can be unsupported or may be supported on any suitable inert carrier such as alumina, silica, alumina-silica, silicon carbide or clay. The catalyst may be disposed on a support by known methods such as impregnation as by incipient wetness techniques, and coprecipitation techniques.

The catalyst may be prepared by conventional means, such as by mixing compounds containing the catalyst components in a liquid solution or slurry and heating; recovering the catalyst precursor from the liquid, drying and calcining.

A preferred catalyst preparation method involves impregnation of an hydroxide or hydrous oxide of component B, especially those hydroxides with layered structures, using a solution of a salt of component A such as a nitrate or acetate. Partially hydrolyzed salts of component A, where small clusters of metal ions form in solution, may also be used. This partial hydrolysis may also be allowed to occur during impregnation by control of pH. The component B hydrous oxide may also be formed on a support before impregnation with component A. Heat treatments to dehyrate hydrous oxides or decompose salts, such as nitrates, may be done at any stage of the preparation, although it is preferred to keep the component B hydrous oxide in as complete a state of hydration as possible before impregnation with component A, so as to encourage higher dispersion of the component A species in the finished catalyst.

This higher dispersion of component A is also favored by co-precipitation of hydroxy carbonate salts or mixed hydrous oxides of components A and B. A carbonate salt of component C might be used for this precipitation, with washing afterward to remove that amount of component C in excess of requirements.

Component C may be added at any stage, although if it is not present as a precipitation agent, it is preferred to add it by impregnation to a partially heat-treated catalyst precursor containing both A and B.

It is preferred that the catalysts be heat treated prior to use in the conversion of low molecular weight alkanes, although this is not required for the present invention. Heat treating has been found to increase the catalyst stability. The catalyst is preferably heat treated to a temperature of between 500°C and 1200°C. Such pretreatment may occur upon startup of the reactor prior to introduction of reactant species into the process.

Products of methane upgrading in accordance with the present invention include ethane, ethylene and higher molecular weight hydrocarbons as well as by-product water, carbon monoxide and carbon dioxide. The conversion of methane by the process of the present invention is as high as from 14.5 percent to 21.1 percent while maintaining reaction selectivity for the formation of higher order hydrocarbons in the range of from 37 percent to 62 percent. This process produces an effective yield of higher order hydrocarbons by upgrading methane of from 6 percent to 13 percent, signficantly higher yields than those obtained by previously disclosed processes and catalysts.

It is envisioned that unconverted methane may be recycled to the reaction so as to increase the overall yield of products by this methane upgrading.

When natural gas comprises a portion of the feed stream, the minor amounts of other low molecular weight alkanes are upgraded in a manner similar to that of methane. Ethane may be converted to butane and propane. If the reaction contemplates recycling the unreacted natural gas, then the portion of the feed stream containing minor amounts of the alkanes ethane, butane, propane and pentane may change, depending on the efficiency of the product recovery apparatus. These alkanes need not be fully removed from the reactor feed stream. The resultant products are substantially higher order hydrocarbons.

The higher order hydrocarbons may be easily transported and have versatile applications in chemical processing as well as uses as fuels. In addition, these higher order hydrocarbons may be further processed to form substantially liquid hydrocarbon products, as for example in accordance with the process for converting ethane to LPG and gasoline and/or aromatics concentrate disclosed in previously-referenced United States Patent 4,100,218 to Chen et al. By this manner an upgraded hydrocarbon, such as ethane upgraded from methane, is subjected to thermal cracking at temperatures of from 815°C to 875°C to produce an olefin-rich effluent which is then cooled to a temperature between 315°C and 650°C and contacted with a crystalline aluminosilicate zeolite so as to produce a liquid hydrocarbon product suitable for use as LPG, gasoline and/or aromatics concentrate.

Other known processes are also available for the conversion of ethane and ethylene to ethanol, ethylene glycol, polyethylene, and other additional chemicals useful as fuels, fuel additives and lubricants. Thus, the process disclosed herein for upgrading low molecular weight alkanes to higher order hydrocarbon products may be integrated into a further process for converting such alkanes to useful chemicals.

The following examples demonstrate the effectiveness of the present invention.

Examples

Catalysts were prepared as described individually below. The catalysts were then used in a process to convert low molecular weight alkanes to higher molecular weight hydrocarbons. Reaction products were measured to determine individual yields and conversion rates.

Catalyst preparation

A comparative catalyst of about thirty-three weight percent lead oxide, PbO, on gamma-alumina, similar to the single component catalyst described by Baerns and Hinsen in German Patent 3,237,079, was prepared by impregnating a solution comprising about 37 grams of lead nitrate, $Pb(NO_3)_2$, in about 45 cc distilled water onto about 50 grams of an alumina support, Norton HSA alumina extrudates. The alumina support had an initial measured surface area of 270 $m^2/gm$. It was necessary to impregnate the support with the impregnating solution twice, drying the support at about 110°C in air between impregnations, so that all the solution was sorbed into the pores of the support. After the second impregnation the support was dried at about 110°C in air, and then further heat treated at about 290°C in air for about six hours and at about 425°C in air for about 16 hours. The so formed catalyst had the shape of about 1/16" diameter by about 6 mm long extrudates and had a measured surface of about 160 $m^2/gm$.

Catalyst 1 comprised about 20 weight percent lead oxide, PbO, combined with magnesium oxide, MgO. This catalyst was prepared by impregnating a solution of about 1.99 grams of lead nitrate, $Pb(NO_3)_2$ in about 8.2 cc distilled water onto about 8 grams of magnesium hydroxide powder, $Mg(OH)_2$. The magnesium hydroxide powder had been prepared by adding concentrated ammonia solution to a solution of magnesium nitrate, $Mg(NO_3)2 \cdot 6H_2O$, in water, heating to about 40°C with stirring for about 20 hours; filtering; then drying at about 110°C for about 20 hours in air.

The adhesive, white solid obtained after adding lead nitrate to magnesium hydroxide was dried for about 20 hours at about 110°C to give a hydrated catalyst precursor. About 6.2 grams of the precursor, having a particle size between about 20 and 35 mesh, was then heat treated in the presence of flowing nitrogen. The heat treatment comprised heating from 100°C to about 300°C over about a four hour period, heating to about 320°C and maintaining that temperature for about 20 hours. The resultant material was a light yellow granular powder, which was further heated under flowing nitrogen for about six hours at about 360°C. This powder had a surface area of about 56 $m^2/gm$ and an approximate composition $Pb_1Mg_{22}O_x$.

Catalyst 2 comprised lead oxide and magnesium oxide on an aluminum oxide support. The catalyst was prepared by impregnating about 85 grams of magnesium hydroxide on an alumina hydrate powder with a solution comprising about 26.5 grams of lead nitrate and about 45 cc of distilled water. The magnesium hydroxide on alumina hydrate powder was prepared by precipitating magnesium hydroxide in the presence of alumina hydrate from a concentrated ammonia solution. The ratio of magnesium to aluminum was about 1:1. After impregnation, the powder was heat treated in a manner similar to that described above for the heat treating of Catalyst 1. The resultant powder was a light yellow-tan material and had an approximate formula $Pb_1\ Mg_{9.7}O_x$—$Al_2O_3$ with a composition of about 55 weight percent $Pb_1\ Mg_{9.7}O_x$ and 45 weight percent $Al_2O_3$.

Catalyst 3 comprised lead oxide, magnesium oxide and zinc oxide on an aluminum oxide support. The catalyst was prepared by impregnating about 168 grams of a mixed magnesium-zinc hydroxide on alumina hydrate powder with a solution comprising about 51 grams of lead nitrate and about 75 cc of distilled water. The mixed magnesium-zinc hydroxide on alumina hydrate powder was prepared by precipitating magnesium hydroxide and hydrous zinc oxide at the same time in the presence of alumina hydrate using concentrated ammonia solution. The ratio of magnesium to zinc to aluminum was about 0.5:0.5:2. After impregnation the powder was heat treated in a manner similar to that described above for catalyst 1. The resultant material was a light yellow powder of approximate composition $Pb_3Mg_8Zn_8O_x$—$Al_2O_3$ with a composition of about 50 weight percent $Pb_3Mg_8Zn_8O_x$ and about 50 weight percent $Al_2O_3$.

Reaction conditions

The oxidation of methane was performed using a commercial grade of methane, about 99.4 percent methane and about 0.62 percent ethane. A methane/oxygen/nitrogen mixture was passed through a quartz tube in an electrically heated furnace. The catalyst material was placed in a tube near the center of the heated zone of the furnace in a bed less than 50 mm long. The remaining heated portion of the tube was filled with fused quartz chips to provide a preheated initial zone and to decrease residence time after the catalyst. A thermocouple extended into the top few millimeters of the catalyst bed to allow direct temperature measurements.

After passing through the reactor ethylene, ethane and methane were separated on a 10' long Porapak QS column in a Hewlett Packard 5711 Gas Chromatograph with flame ionization detection. Relative molar responses according to Dietz were used to obtain the ratio of hydrocarbon products to methane from the flame detector area. These are close to the unit response per carbon atom rule which applies to flame ionization of hydrocarbons. Oxygen, nitrogen, methane, carbon dioxide and carbon monoxide were determined with a Fisher 1200 Gas Chromatograph where the lighter gases were separated on molecular sieve 13× in parallel with a column for separating carbon dioxide and heavier hydrocarbons, especially $C_3$ and $C_4$ hydrocarbons.

The catalysts were all heat treated in the reactor prior to methane conversion. The oxygen/nitrogen portion of the feed gas was passed over the heated catalyst at about 180 cc/minute. Temperature rise was gradual, heating from room temperature to about 700°C over a period of about 20 hours. The final temperature, about 700°C, was maintained for about three hours prior to the introduction of the methane portion of the feed into the reactor.

Eleven runs were performed to examine the effectiveness of the catalysts for methane dimerization in

accordance with this invention when contrasted to previously disclosed catalysts and when no catalyst was used. An additional run was performed to demonstrate the effectiveness of the catalysts disclosed herein for ethane coupling.

Examples 1—10

In control runs, 1 and 2, about 1.08 grams of alumina chips were used to form about a 35 mm long reactor bed. Thus, no catalyst was present in these runs. The feed gas comprised methane/nitrogen/oxygen in a ratio of about 1:2.78:0.20. The feed rate was about 180 cc/minute NTP. Oxygen and methane conversions were both very small. Yields of higher order hydrocarbons were near those expected if the higher order hydrocarbons present as impurities in the feed passed through unconverted.

Runs 3 and 4 utilized the comparative catalyst described above to convert methane to higher molecular weight hydrocarbons. About 1.04 grams of lead oxide-aluminum oxide was used to form about a 15 mm long bed in the reactor. The feed gas comprising methane/nitrogen/oxygen in a ratio of about 1:2.79:0.19 was fed into the reactor at about 182 cc per minute NTP. The selective formation of higher molecular weight hydrocarbons and conversion yields were close to those found by Baerns and Hinsen as reported for a similar catalyst in German Patent No. 3,237,079. The production rate for dimers of methane at about 721°C was seen to be about 0.117 grams methane per gram catalyst-hour.

Runs 5 and 6 utilized the above-described Catalyst 1. The about 0.8 grams of lead oxide and magnesium oxide catalyst formed about a 50 mm long bed in the reactor. The feed gas comprising methane/nitrogen/oxygen in a ratio of about 1:2.71:0.21 was fed at about 184 cc per minute through the reactor. The selective formation of higher molecular weight hydrocarbons and the percent conversion of methane were significantly higher than those for the comparative catalyst shown in Runs 3 and 4. The production of dimers of methane at about 717°C was about 0.228 grams methane per gram catalyst hour, nearly twice that of the comparative case in Run 3.

Runs 7 and 8 were performed utilizing Catalyst 2. About 1.48 grams of the lead oxide and magnesium oxide on an aluminum oxide support formed about a 50 mm long bed in the reactor. The feed gas of methane/nitrogen/oxygen in a ratio of about 1:2.71:0.22, respectively, was fed at about 179 cc per minute. Higher molecular weight hydrocarbon selectivities and percent conversion were higher than those for the comparative catalyst shown in Runs 3 and 4, with the selective formation of ethylene particularly high.

Run 9 utilized catalyst 3. About 1.40 grams of the lead oxide, magnesium oxide and zinc oxide on an aluminum oxide support formed about a 50 mm long bed in the reactor. The feed gas was methane/nitrogen/oxygen in a ratio of about 1:2.68:0.21, and was fed at a rate of about 183 cc per minute. Significant yields of higher molecular weight hydrocarbons, especially ethylene, and conversion rates for methane were realized with this catalyst.

Run 10 utilized catalyst 3 along with a measurable amount of water vapor in the feed stream. The feed gas in run 10 was methane/nitrogen/oxygen/water vapor in a ratio of about 1:2.68:0.21:0.4. This feed gas passed through the reactor at a rate of about 183 cc per minute. Water vapor was equivalent to about 40 cc per minute NTP extra diluent in this run. Again, high yields of higher molecular weight hydrocarbons were realized, especially ethylene.

Data for each of these ten runs is presented in Table 1. As can be seen from the table, the process in accordance with the present invention produces high yields of higher order hydrocarbons, especially ethane and ethylene as well as significant conversion rates for methane. The combined selectivity for higher order hydrocarbons and high conversion rate of methane result in an improved process for upgrading methane.

Example 11

This example demonstrates the upgrading of methane utilizing a catalyst disclosed herein and operating in a cyclic process, as is known in this art, cycling between a reaction step and a catalyst re-oxidation step.

A reactor and catalyst as used in Examples 7 and 8 above were utilized in the following manner:

The reactor was heated to about 720°C during which time a stream of nitrogen gas flowed through the reactor. Oxygen was then added to the nitrogen stream to fully oxidize the catalyst. The oxygen stream was stopped and, about one minute later, methane was introduced to the reactor feed stream. Methane comprised about thirty volume percent of the reactor feed stream.

After about 60 seconds, the exit stream was sampled and an ethane-ethylene mixture equivalent to about 1.5 weight percent of the carbon in the stream was found along with methane. This ethane-ethylene mixture had about 40 weight percent ethylene in it, thus ethylene was equivalent to about 0.6 weight percent of the carbon fed. However, when the reactor feed stream was sampled prior to introduction into the reactor, the only two-carbon impurity found was ethane, equivalent to about 1.2 weight percent of the feed carbon. Thus, the amount of higher order hydrocarbons was improved by passage over the catalyst, and much of the impurity ethane in the feed stream was converted to ethylene.

Example 12

A run was also made using a commercial grade of ethane, having more than 99 weight percent ethane purity and less than 0.1 weight percent ethylene and $C_3$ or higher order hydrocarbon impurities, to examine

the effectiveness of the catalysts disclosed herein for coupling low molecular weight alkanes other than methane. The catalyst used in this example was catalyst 2, lead oxide and magnesium oxide on an aluminum oxide support, as described hereinabove. About 0.75 grams of this catalyst was mixed with 12—20 mesh fused quartz chips and disposed in a quartz tube to make a reaction bed about 75 mm long. The remaining heated portion of the tube was filled with fused quartz chips. A reactor feed stream comprising ethane/oxygen/nitrogen in mole ratios of about 1.0/0.2/1.2 was fed at about 170 cc/minute NTP through the reactor whereupon the temperature in the catalyst bed rose from about 520°C to about 614°C. As shown in Table 2, ethylene and $C_3$/$C_4$ hydrocarbons form a substantial portion of the synthesized products. Thus, the process and catalysts of this invention are shown to be effective for the overall upgrading of low molecular weight alkanes to higher order hydrocarbons.

TABLE 1
Methane conversion

| Run number | Catalyst composition | Reaction temperature (°C) | Methane conversion (percent) | Percent methane converted to ethylene | Percent methane converted to ethane | Percent methane converted to $CO_2$ | Higher order hydrocarbon selectivity (percent) | Overall methane conversion to higher order hydrocarbons (percent) |
|---|---|---|---|---|---|---|---|---|
| 1 | None | 720 | 0.05 | 0 | 1.3 | 0 | 0 | 0 |
| 2 | None | 770 | 0.4 | 0.1 | 1.5 | 0 | 0 | 0 |
| 3 | $PbO/Al_2O_3$ | 693 | 16.2 | 2.1 | 4.1 | 10.0 | 38 | 6.2 |
| 4 | $PbO/Al_2O_3$ | 721 | 17.2 | 2.6 | 4.1 | 10.5 | 39 | 6.7 |
| 5 | $Pb_1Mg_{22}O_x$ | 686 | 18.1 | 2.5 | 7.3 | 8.4 | 54 | 9.8 |
| 6 | $Pb_1Mg_{22}O_x$ | 717 | 21.1 | 4.0 | 9.1 | 8.0 | 62 | 13.1 |
| 7 | $Pb_1Mg_{9.7}O_x/Al_2O_3$ | 732 | 17.6 | 3.0 | 5.1 | 9.5 | 46 | 8.1 |
| 8 | $Pb_1Mg_{9.7}O_x/Al_2O_3$ | 756 | 18.2 | 4.1 | 4.7 | 9.5 | 48 | 8.7 |
| 9 | $Pb_3Mg_8Zn_8O_x/Al_2O_3$ | 702 | 14.5 | 2.8 | 2.9 | 8.8 | 39 | 5.7 |
| 10 | $Pb_3Mg_8Zn_8O_x/Al_2O_3 \cdot H_2O$ | 683 | 16.2 | 3.0 | 3.1 | 10.1 | 37 | 6.0 |

TABLE 2
Ethane conversion

| Run number | Catalyst composition | Reaction temperature (°C) | Ethane conversion (percent) | Percent ethane converted to ethylene | Percent ethane converted to propane, propylene and butane | Percent ethane converted to $Co_2$, $CH_4$ and CO | Overall ethane selectivity to ethylene and higher order hydrocarbons (percent) |
|---|---|---|---|---|---|---|---|
| 12 | $Pb_1Mg_{9.7}O/Al_2O_3$ | 613 | 13 | 3.5 | 0.85 | 8.7 | 33 |

**Claims**

1. A process for the conversion of low molecular weight alkanes of the formula $C_nH_{2n+2}$ wherein n ranges from 1 to 5 to higher order hydrocarbons comprising contacting said low molecular weight alkanes at a reaction temperature of from 500°C to 1000°C with a catalyst of the formula

$$A_a B_b C_c O_x$$

wherein
A is Pb, Bi, Sn, Sb, Tl, In, Mn, Cd, Ge or mixtures thereof;
B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and
x is the number of oxygens needed to fulfill the valence requirements of the other elements.

2. The process in accordance with Claim 1 wherein said low molecular weight alkanes comprises predominantly methane.

3. The process in accordance with Claim 2 wherein said higher order hydrocarbons are predominantly ethane and ethylene.

4. The process in accordance with Claim 1 wherein said contacting said low molecular weight alkanes occurs in the presence of gaseous oxygen.

5. The process in accordance with Claim 1 wherein said low molecular weight alkanes comprise from 15 volume percent to 100 volume percent of a reaction feed stream.

6. The process in accordance with Claim 4 wherein said gaseous oxygen comprises from zero volume percent to about fifty volume percent of a reaction feed stream.

7. The process in accordance with Claim 1 wherein a diluent gas is present.

8. The process in accordance with Claim 1 wherein A is Pb.

9. The process in accordance with Claim 1 wherein B is selected from Mg, Ca, Zn and mixture thereof.

10. The process in accordance with Claim 1 wherein said catalyst is supported on an inert carrier.

11. A catalyst, which has been heat treated at a temperature of between 500°C and 1200°C for the conversion of methane to higher order hydrocarbons of the formula

$$A_a B_b C_c O_x$$

wherein
A is Pb, Bi, Sn, Sb, Tl, In, Mn, Cd, Ge or mixtures thereof;
B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and
x is the number of oxygens needed to fulfill the valence requirements of the other elements.

12. The catalyst in accordance with Claim 11 wherein A is selected from Pb, Bi, Sn, Sb, Mn and mixtures thereof.

13. The catalyst in accordance with Claim 11 wherein A is Pb.

14. The catalyst in accordance with Claim 11 wherein B is selected from Mg, Ca, Zn and mixtures thereof.

15. A process as claimed in any of claims 1 to 10 for upgrading low molecualr weight alkanes to substantially liquid hydrocarbon products, which process comprises
contacting low molecular weight alkanes at a reaction temperature of from 500°C to 1000°C in the presence of a catalyst of the formula

$$A_a B_b C_c O_x$$

wherein
A is Pb, Bi, Sn, Sb, Tl, In, Mn, Cd, Ge or mixtures thereof;
B is Mg, Ca, Sr, Ba, Zn, La, Ce, Sc, Y, Cu, Ni, Ti, Zr, Co, V or mixtures thereof;
C is Li, Na, K, Rb, Cs or mixtures thereof; and
wherein
a is from 0.1 to 100;
b is from 0.1 to 100;
c is from 0.0 to 100; and
x is the valence requirements of the other elements so as to synthesize a higher order hydrocarbon product effluent; and
characterised in that it includes the further step of dehydrogenating the higher order hydrocarbon

and/or contacting the higher order hydrocarbon with a catalyst to yield a substantially liquid hydrocarbon product.

**Patentansprüche**

1. Verfahren zur Umwandlung von Alkanen mit niedrigem Molekulargewicht, mit der Formel $C_nH_{2n+2}$, worin n im Bereich von 1 bis 5 liegt, in Kohlenwasserstoffe höherer Größenordnung, durch Kontakt der Alkane mit niedrigem Molekulargewicht bei einer Reaktionstemperatur von 500°C bis 1000°C mit einem Katalysator der Formel

$$A_a B_b C_c O_x$$

worin
A Pb, Bi, Sn, Sb, Tl, In, Mn, Cd, Ge oder Gemische davon ist;
B Mg, Ca, Sr, Ba, Zn, Le, Ce, Sc, Y, Cu, Ni, Ti, Zr, Co, V oder Gemische davon ist;
C Li, Na, K, Rb, Cs oder Gemische davon ist; und
worin
a 0,1 bis 100 ist;
b 0,1 bis 100 ist;
c 0,0 bis 100 ist; und
x die Anzah der Sauerstoffe ist, die zur Erfüllung der Wertigkeitserfordernisse der anderen Elemente notwendig sind.

2. Verfahren nach Anspruch 1, worin die Alkane mit niedrigem Molekulargewicht vorwiegend Methan umfassen.

3. Verfahren nach Anspruch 2, worin die Kohlenwasserstoffe höherer Größenordnung vorwiegend Ethan und Ethylen sind.

4. Verfahren nach Anspruch 1, worin der Kontakt der Alkane mit niedrigem Molekulargewicht in der Anwesenheit von gasförmigem Sauerstoff erfolgt.

5. Verfahren nach Anspruch 1, worin die Alkane mit niedrigem Molekulargewicht 15 Vol.-% bis 100 Vol.-% eines Reaktions-Beschickungsstromes betragen.

6. Verfahren nach Anspruch 4, worin der gasförmige Sauerstoff von Null Vol.-% bis etwa 50 Vol.-% eines Reaktions-Beschickungsstromes beträgt.

7. Verfahren nach Anspruch 1, worin ein Verdünnungsgas vorhanden ist.

8. Verfahren nach Anspruch 1, worin A Pb ist.

9. Verfahren nach Anspruch 1, worin B ausgewählt ist aus Mg, Ca, Zn und Gemischen davon.

10. Verfahren nach Anspruch 1, worin der Katalysator auf einem inerten Träger getragen wird.

11. Katalysator, der einer Wärmebehandlung bei einer Temperatur von zwischen 500°C und 1200°C unterzogen wurde, zur Umwandlung von Methan in Kohlenwasserstoffe höherer Ordnung, mit der Formel

$$A_a B_b C_c O_x$$

worin
A Pb, Bi, Sn, Sb, Tl, In, Mn, Cd, Ge oder Gemische davon ist;
B Mg, Ca, Sr, Ba, Zn, La, Ce, Sc, Y, Cu, Ni, Ti, Zr, Co, V oder Gemische davon ist;
C Li, Na, K, Rb, Cs oder Gemische davon ist; und
worin
a 0,1 bis 100 ist;
b 0,1 bis 100 ist;
c 0,0 bis 100 ist; und
x die Anzahl der Sauerstoffe ist, die zur Erfüllung der Wertigkeitserfordernisse der anderen Elemente notwendig sind.

12. Katalysator nach Anspruch 11, worin A ausgewählt ist aus Pb, Bi, Sn, Sb, Mn und Gemischen davon.

13. Katalysator nach Anspruch 11, worin A Pb ist.

14. Katalysator nach Anspruch 11, worin B ausgewählt ist aus Mg, Ca, Zn und Gemischen davon.

15. Verfahren nach einem der Ansprüche 1 bis 10 zur Aufwertung von Alkanen mit niedrigem Molekulargewicht in im wesentlichen flüssige Kohlenwasserstoffprodukte, welches den Kontakt von Alkanen mit niedrigem Molekulargewicht bei einer Reaktionstemperatur von 500°C bis 1000°C in der Anwesenheit eines Katalysators der Formel

$$A_a B_b C_c O_x$$

umfaßt,
worin
A Pb, Bi, Sn, Sb, Tl, In, Mn, Cd, Ge oder Gemische davon ist;
B Mg, Ca, Sr, Ba, Zn, La, Ce, Sc, Y, Cu, Ni, Ti, Zr, Co, V oder Gemische davon ist;
C Li, Na, K, Rb, Cs oder Gemische davon ist; und

worin
a 0,1 bis 100 ist;
b 0,1 bis 100 ist;
c 0,0 bis 100 ist; und
x den Wertigkeitserfordernissen der anderen Elemente entspricht, um einen Kohlenwasserstoff-Produkt-Abstrom höherer Größenordnung zu synthetisieren,
dadurch gekennzeichnet, daß es die weitere Stufe der Dehydragenierung der Kohlenwasserstoffe höherer Größenordnung und/oder den Kontakt der Kohlenwasserstoffe höherer Größenordnung mit einem Katalysator umfaßt, um ein im wesentlichen flüssiges Kohlenwasserstoffprodukt zu ergeben.

**Revendications**

1. Procédé de conversion d'alcanes de formule $C_nH_{2n+2}$, n valant de 1 à 5, de faible poids moléculaire, en hydrocarbures d'ordre plus élevé par mise en contact desdits alcanes de faible poids moléculaire, à une température de réaction allant de 500°C à 1000°C, avec un catalyseur de formule

$$A_a B_b C_c O_x$$

où
A est Pb, Bi, Sn, Sb, Tl, In, Mn, Cd, Ge ou leurs mélanges;
B est Mg, Ca, Sr, Ba, Zn, La, Ce, Sc, Y, Cu, Ni, Ti, Zr, Co, V ou leurs mélanges;
C est Li, Na, K, Rb, Cs ou leurs mélanges; et
où
a vaut de 0,1 à 100;
b vaut de 0,1 à 100;
c vaut de 0,0 à 100; et
x est le nombre d'oxygènes nécessaires pour remplir les besoins en valence des autres éléments.

2. Procédé selon la revendication 1, dans lequel lesdits alcanes de faible poids moléculaire comprennent principalement le méthane.

3. Procédé selon la revendication 2, dans lequel lesdits hydrocarbures d'ordre plus élevé sont principalement l'éthane et l'éthylène.

4. Procédé selon la revendication 1, dans lequel ladite mise en contact desdits alcanes de faible poids moléculaire se fait en présence d'oxygène gazeux.

5. Procédé selon la revendication 1, dans lequel lesdits alcanes de faible poids moléculaire constituent de 15 volumes pour cent à 100 volumes pour cent du flux d'alimentation de la réaction.

6. Procédé selon la revendication 4, dans lequel ledit oxygène gazeux constitue de zéro volume pour cent à environ cinquante volumes pour cent du flux d'alimentation de la réaction.

7. Procédé selon la revendication 1, dans lequel un gaz de dilution est présent.

8. Procédé de la revendication 1, dans lequel A est Pb.

9. Procédé selon la revendication 1, dans lequel B est choisi parmi Mg, Ca, Zn et leurs mélanges.

10. Procédé selon la revendication 1, dans lequel ledit catalyseur est porté par un support inerte.

11. Catalyseur, qui a été traité à une température située entre 500°C et 1200°C, pour la conversion du méthane en hydrocarbures d'ordre plus élevé, de formule

$$A_a B_b C_c O_x$$

où
A est Pb, Bi, Sn, Sb, Tl, In, Mn, Cd, Ge ou leurs mélanges;
B est Mg, Ca, Sr, Ba, Zn, La, Ce, Sc, Y, Cu, Ni, Ti, Zr, Co, V ou leurs mélanges;
C est Li, Na, K, Rb, Cs ou leurs mélanges; et
où
a vaut de 0,1 à 100;
b vaut de 0,1 à 100;
c vaut de 0,0 à 100; et
x est le nombre d'oxygènes nécessaires pour remplir les besoins en valence des autres éléments.

12. Catalyseur selon la revendication 11, dans lequel A est choisi parmi Pb, Bi, Sn, Sb, Mn et leurs mélanges.

13. Catalyseur selon la revendication 11, où A est Pb.

14. Catalyseur selon la revendication 11, où B est choisi parmi Mg, Ca, Zn et leurs mélanges.

15. Procédé tel que revendiqué dans les revendications 1 à 10 pour la valorisation d'alcanes de faible poids moléculaire en produits hydrocarbonés substantiellement liquides, procédé qui comporte le fait de mettre en contact des alcanes de faible poids moléculaire à une température de réaction allant de 500°C à 1000°C en présence d'un catalyseur de formule

$$A_a B_b C_c O_x$$

où
A est Pb, Bi, Sn, Sb, Ti, In, Mn, Cd, Ge ou leurs mélanges;

B est Mg, Ca, Sr, Ba, Zn, La, Ce, Sc, Y, Cu, Ni, Ti, Zr, Co, V et leurs mélanges;
C est Li, Na, K, Rb, Cs ou leur mélanges; et

où

a vaut de 0,1 à 100;
b vaut de 0,1 à 100;
c vaut de 0,0 à 100; et
x est le besoin en valence des autres éléments pour synthétiser comme effluent un produit hydrocarboné d'ordre supérieur; et

caractérisé en ce qu'il comporte l'étape suivante consistant à déshydrogéner l'hydrocarbure d'order plus élevé et/ou à mettre en contact l'hydrocarbure d'ordre plus élevé avec un catalyseur pour obtenir un produit hydrocarboné substantiellement liquide.